# EUROPEAN PATENT SPECIFICATION

(11) **EP 3 452 027 B1**
(45) Date of publication and mention of the grant of the patent: **16.09.2020**
(21) Application number: 17723650.2
(22) Date of filing: 02.05.2017
(51) Int. Cl.: A61K 31/122, A61K 45/06, A01K 63/00, A01K 63/04, A01K 71/00, A01K 73/00, A01K 74/00, A23K 50/80, A61P 33/14

(54) **USE OF NOOTKATONE TO TREAT SEA LICE**
VERWENDUNG VON NOOTKATON ZUR BEHANDLUNG VON SEELÄUSEN
UTILISATION DE NOOTKATONE POUR TRAITER LES POUX DE MER

(30) Priority: 02.05.2016 US 201662330391 P
(43) Date of publication of application: 13.03.2019
(73) Proprietor: Evolva SA, 4153 Reinach (CH)
(72) Inventor: GOLDSMITH, Neil, 2800 Delemont (CH)
(74) Representative: Forrest, Stuart
(86) International application number: PCT/EP2017/060431
(87) International publication number: WO 2017/191138

(56) References cited:
- WO-A1-2014/031790
- WO-A1-2015/021534
- WO-A1-2015/155293

## Description

### BACKGROUND OF THE INVENTION

### Field of the Invention

This disclosure relates to nootkatone-containing compositions for use in treating and preventing sea louse infection in fish.

### Description of Related Art

Crustacean parasites of fish, amphibians and invertebrates (including larger crustaceans such as mollusks, shrimps and crabs) include copepods, branchiurans, tantlocarids, amphipoda, isopods and rhizosephalans. Crustacean ectoparasites (external parasites) of fish feed on mucus, epidermal tissue, and blood of hosts. They have been reported as inducing negative effects on host activity, shoaling behaviour, growth rates, damaging host integument (via attachment, feeding, injection of enzymes), eliciting inflammatory immune responses, eliciting cortisol-mediated stress responses, acting as vectors and causing secondary infections.

The *Argulidae* family of fish lice are one of several families of copepod crustaceans parasisitic to fishes. There are hundreds of species of fish lice, with current estimates of 175 species in 1 genus, the *Argulidae.* An example species is *Argulus foliaceus*, often referred to as the common fish louse, which is considered to be one of the most widespread crustacean ectoparasites of freshwater fish in the world. *Argulus foliaceus* is 5mm by 7mm when fully grown and has been recorded on virtually every freshwater fish species within its range (Walker et al. 2007."Size matters: stickleback size and infection with Argulus foliaceus" Crustaceana 80(11), 1397-1401). Notable food, sport and ornamental hosts include salmon, trout, sunfish, carp, bream, goldfish, pike, perch, roach, rudd, catfish, zander, tench, frogs and toads (Pasternak et al, 2000. "Life history characteristics of Argulus foliaceus L. (Crustacea: Branchiura) populations in Central Finland". Annales Zoologici Fennici 37(1), 25-35). Common symptoms of infestation include inflammation of the skin, open hemorrhaging wounds, anemia, loss of appetite, reduced growth, increased production of mucus, loss of scales, and corrosion of the fins. On larger fish, the parasite load in entire populations of host fish has been reported in the hundreds and even 1000 lice per fish. Such heavy infestations in commercial fish stocks (such as for food, sport or breeding of ornamental fish) has been reported in the industry as resulting in large financial losses and temporary closure of the aquaculture to allow for quarantine and thorough attempts at treatment.

Fish lice in the genus *Argulus* attach to hosts using two suction cups in the head and hooked appendages on the body. The host skin is pierced using a stylet to feed on the blood and digestive enzymes injected into the flesh. These wounds often become infected with bacteria and fungi, but the fish louse is also acknowledged as a specific vector for *Skrjabillanidae* nematodes, viruses (such as *Rhabdovirus carpio*), flagellates, bacteria and fungi. Although Branchiurams are generally freshwater ectoparasites of fishes, *Argulus* infestations have been reported to cause mortality in farmed marine salmonid stocks in Chile and Canada.

There are hundreds of species of sea lice, with current estimates of 557 species in 37 genera, but most are classified within two genera, *Lepeophtheirus* (162 species) and *Caligus* (268 species). Sea lice are all copepods within the order Siphonostomatoida, the Caligidae.

Of overriding interest to marine fish farmers, *Lepeophtheirus salmonis* (salmon louse) is a sea louse that parasitizes numerous salmon species including the widely farmed Atlantic salmon (*Salmo salar*). However, the salmon louse can parasitize other salmonids to varying degrees, including all species of Pacific salmon, brown trout, sea trout (*Salmo trutta*), and Arctic char (*Salvelinus alpinus*). *Lepeophtheirus pectoralis* is a sea louse that uses salmon and flatfish, including plaice and European flounder, as hosts. *Caligus elongatus* parasitizes over 80 species of marine fish, including salmon, lumpfish, saithe, pollock, sea trout, herring, Atlantic cod, and char.

A sea louse egg hatches into nauplius I, which molts into a second naupliar stage. Both naupliar stages are non-feeding, depending on yolk reserves for energy, and adapted for swimming. The copepodid stage is the mobile infectious stage when the sea louse nauplius searches for an appropriate host, likely by chemo- and mechanosensory clues assisted by sea currents, salinity, light, and other factors. The preferred settlement of sea lice on fish occurs in areas with the least hydrodynamic disturbance, particularly the back of the head, on fins, gills and other relatively sheltered areas. Adult females, being larger, occupy relatively flat but sheltered body surfaces on the posterior ventral and dorsal midlines and may actually out-compete pre-adults and males at these sites.

Sea lice (copepodids) attach to a suitable host and feed for a period of time prior to molting to the chalimus I stage. Sea lice continue their development through a further three additional chalimus stages each separated by a molt. Sea lice attach to the host by a structure referred to as the frontal filament. With exception of a short period during the molt, the pre-adult and adult stages are mobile on the fish and, in some cases, can move between fish, such as between host fish in the same fish farm, or between host farmed fish and wild fish species, or between host farmed fish and host/carrier fish such as commensal or mutualistic fish (including cleaner fish), or indirectly to either of these via a solid surface found in a fish farm such as boats, baths, nets, floats, filters, pumps or tools for rearing and harvesting fish.

Sea lice cause physical and enzymatic damage at their sites of attachment and feeding which results in abrasion-like lesions. These lesions are open wounds on the fish capable of acting as entry points for disease and infection. Even after the fish are harvested and any remaining sea lice die and fall away, the lesions remain on the fish and may result in the fish meat having greatly reduced economic value.

Ectoparasitic infections of fish by crustaceans cause a generalized chronic stress response in fish, possibly because feeding and attachment changes host mucus consistency and causes epithelial damage, which results in blood and fluid loss, electrolyte changes, and cortisol release. Whilst the release of stress hormones in the host is likely in part due to the above suggested mechanism and the physical pain of the lesion, there is also evidence that some ectoparasitic crustacean species introduce active agents into their host during attachment or feeding. For example, *Lepeophtheirus salmonis* secrete large amounts of trypsin into their host's mucus, presumably to assist in feeding and digestion. The systemic introduction of such a broad specificity endopeptidase into host fish induces poorly understood adverse reactions. In the case of Pacific salmon, Coho, chum, and pink salmon (O. *kisutch,* O. *keta,* and O. *gorbuscha,* respectively), inflammation and strong immune responses are observed in tissues to which L. *salmonis* has attached. Such immune responses by the host fish can lead to successful rejection of the parasite within the first week of infection, but not always. Indeed, some sea lice secretions have been shown to contain prostaglandin E2 (a suppressor of T cell receptor signaling), and CYP P450s and serpins (another type of defensive enzyme) were down-regulated in host fish, especially in skin tissue, after infection. It is postulated that the downregulation of the host immune response not only leads to subsequent infectious diseases, but also results in reduced growth and performance.

The ability of an ectoparasitic crustacean (such as fish lice and sea lice) species to parasitize across host species means that there is considerable concern that fish stocks on an economic scale (such as fish farms) may be infecting wild fish populations in the same waters. Indeed, there have been reports that increased rates of sea louse infection on wild fish have been found close to areas of intensive fish farming.

Current means for dealing with ectoparasitic crustaceans of fish (such as sea lice) include bathing, spraying or wiping fish and/or aquaculture device surfaces (such as boats, nets, floats, tools, etc.) with topical disinfectants such as hydrogen peroxide (300-500 mg/L for 20 min) which causes sea lice to detach from the treated surface or fish. However, detached ectoparasitic crustaceans (such as sea lice) can remain infectious and capable of reattaching to fish and reinitiating an infection. Further, hydrogen peroxide decomposition can be toxic to fish and to operators.

Another approach is to use drugs to treat infections of ectoparasitic crustaceans of fish. For example, avermectins are used as in-feed treatments to kill sea lice. The first avermectin used was ivermectin, but it is toxic to some fish, causing sedation and central nervous system depression. Emamectin benzoate has a greater safety margin on fish and kills sea lice at both chalimus and mobile stages. However, resistance to avermectins has been noted in *Chalimus rogercresseyi* in Chile and *L. salmonis* on North Atlantic fish farms, likely due to prolonged use leading to up-regulation of P-glycoprotein.

Other drugs include organophosphates, which are acetylcholinesterase inhibitors that cause paralysis leading to death of ectoparasitic crustaceans of fish (such as sea lice). The use of dichlorvos to treat sea lice has been largely superseded by azamethiphos in Europe in response to safety concerns. Azamethiphos is water-soluble and broken down relatively quickly in the environment, but resistance to organophosphates has been reported due to selection pressures for new acetylcholinesterase variants not inhibited by organophosphates.

Further, pyrethroids are direct stimulators of sodium channels in neuronal cells, inducing rapid depolarization and spastic paralysis leading to death. Pyrethroids are only slowly absorbed by the host and rapidly metabolized once absorbed. Cypermethrin and deltamethrin are commonly used to control sea lice but resistance to pyrethroids has been reported and is believed to be due to a mutation leading to a structural change in the sodium channel.

Therefore, in light of the current challenges to controlling ectoparasitic crustaceans of fish (such as sea lice and fish lice), there is a growing need for effective, environmentally friendly compositions and methodologies to prevent and treat ectoparasitic crustacean infection of fish in and around aquaculture facilities.

WO 2015/021534 A1 discloses a limonoid composition for the prevention and control of marine ectoparasites on fish. Said composition comprises at least one limonoid selected from the group consisting of azadirachtin, salannin, meliantriol, karanjin, and nimbin or is an extract from Neem, Milletia pinnata, or Azadirachta indica. Said composition may be deposited on a polymer which is in the form of a net or cage. Alternatively, said composition may be incorporated into a fish feed.

WO 2015/155293 A1 discloses Quillaja Saponaria saponins or a composition comprising Quillaja Saponaria saponins for use in the prevention and/or treatment of an ectoparasitic infection or infestation in fish. The document also discloses a method for the manufacture of a fish feed comprising Quillaja Saponaria saponins.

WO 2014/031790 discloses pest control compositions and, in particular, pest repellent and pesticidal compositions containing nootkatone and/or a derivative or analog thereof, alone or combination with one or more active ingredients are provided. Methods of formulating and using the compositions are also provided.

### SUMMARY OF THE INVENTION

Provided herein are effective natural compositions for use in methods to treat and prevent a crustacean ectoparasite infection of fish species.

In a first aspect, the invention provides a composition for use in treating or preventing an infection of an ectoparasitic crustacean
(such as fish lice or sea lice) on a fish including an effective
concentration of nootkatone and an additive. In one embodiment of the first aspect, the composition is formulated for topical application. In one embodiment of the first aspect, the additive is an antibiotic or an analgesic. In another embodiment of the first aspect, the composition is formulated for ingestion by the fish. When formulated for ingestion by the fish, the additive can be a cereal, a byproduct from cereal, protein or crude protein, an oil and/or fat, a mineral, an antioxidant, a vitamin, or mixtures thereof. In another embodiment of the first aspect, the composition includes an additional active ingredient.

In one embodiment, the invention provides a composition for use in treating an infection of an ectoparasitic crustacean (such as fish lice or sea lice) on a fish that includes an effective concentration of nootkatone. The nootkatone composition is formulated for dispersion within a pool of water.

Also disclosed is a method of preventing crustacean ectoparasite attachment to or for detaching a crustacean ectoparasite from a fish, the method including applying an effective concentration of nootkatone to the fish. In such a method, the nootkatone is applied at least one of directly and indirectly. Compositions used in such methods may be directly applied by at least one of topical or by ingestion. In other methods, the fish is within a body of water and indirect application comprises treatment of the body of water with a nootkatone-containing composition. In some cases, the effective concentration for fish that are less than about 3 cm long is between about 0.1 and about 5 ppm. The fish may be exposed to the effective concentration for less than about 30 minutes.

In some embodiments, the invention provides a fish net including a coating of nootkatone.

Also provided is a fish tank including a nootkatone-containing liner.

Some of the disclosed compositions for preventing crustacean ectoparasite infection of a fish include nootkatone and an additional active ingredient.

Also disclosed is a method of reducing the frequency of damaged fish meat from farmed fish resulting from physical, enzymatic, or stress damage caused by crustacean ectoparasite, the method including applying a nootkatone comprising composition to fish, water, or a fish farm surface.

In some embodiments, the invention provides a composition for use in a method of treating an infection of an ectoparasitic crustacean on a fish wherein the composition reduces rates of crustacean ectoparasite infection and reduces incidents of bacterial or viral infection caused by crustacean ectoparasite infection.

Optionally, the composition comprising nootkatone is applied every 17 to 72 days.

In a second aspect, the invention provides a fish food including nootkatone. In one embodiment the fish food includes between 0.1 and 10 percent by weight
nootkatone.

In a third aspect, the invention provides a composition for use in a method of reducing an ectoparasitic crustacean infection on farmed fish, the method including treating a cleaner fish species that cohabitates with the farmed fish with a composition comprising nootkatone. In one embodiment of the tenth aspect, the cleaner species is from the family Labridae.

The composition may be used in a method according to any of the previous aspects where the host fish species is selected from salmon, trout, tilapia, catfish, flatfish and carps. Methods and compositions according to any of the previous aspects are contemplated where the composition comprising nootkatone is a solution, an emulsion, a powder, a granule, or a pellet applied to a bath, dip, spray, or a wipe.

Methods and compositions according to any of the previous aspects that use topical compositions are contemplated where the topical composition is applied to at least one of skin, gills, eyes, mouth, scales, or fins of the fish.

Methods and compositions according to any of the previous aspects when fish are present are contemplated where the fish are present for at least one day in water with nootkatone present at a concentration of at least about 800 ppm. Optionally, the fish are present for at least five days in water with nootkatone present at a concentration of at least about 100 ppm.

In some aspects, the nootkatone-comprising composition can be applied to the fish or surface at a concentration of about 10 to about 50 mg/L. In one aspect, the nootkatone-comprising composition can be applied to the fish or surface at a concentration of about 15 to about 20 mg/L. In another aspect, the nootkatone-comprising composition can be applied to the fish or surface at a concentration of about 13.5 mg/L. Fish less than about 3 cm long can be exposed to between about 0.1 and about 5 ppm for less than 30 minutes for an effective treatment.

In a fourth aspect, the invention provides a composition for use in a method of preventing an ectoparasitic crustacean infection of fish, the method including application of a nootkatone composition to the surface of aquaculture equipment. In one embodiment of the fourth aspect, the aquaculture equipment includes one or more of a boat, a bath, a net, a fish tank liner, a float, a hose, a tarpaulin, a skirt, a filter, a pump, or a tool.

A composition for use in a method of farming fish is contemplated in which the fish are separated by year classes and any transfer into a new fish net or cage be performed only after contact of the fish or the net or cage with nootkatone according to any of the preceding aspects.

Optionally, nootkatone is applied or impregnated into the fibers of fish nets suitable for use in fish farms, including open net fish farms, such as boats, nets, floats, filters, and/or tools.

A composition for use in a method of reducing the transfer of crustacean ectoparasite from fish farms into a population of wild fish or shellfish by treating the farmed fish, commensal or mutualist fish, or fish farm surfaces according to any of the preceding aspects is also contemplated.

Optionally, in any of the preceding aspects or embodiments, the composition comprises nootkatone ex valencene.

In another embodiment of any of the preceding aspects or embodiments, the effective concentration of nootkatone is about 0.1 to about 50 mg/L, or about 15 to about 20 mg/L, or about 13.5 mg/L.

These and other features and advantages of the present invention will be more fully understood from the following detailed description of the invention taken together with the accompanying claims. It is noted that the scope of the claims is defined by the recitations therein and not by the specific discussion of features and advantages set forth in the present description.

### DESCRIPTION OF DRAWINGS

Figure 1 illustrates a biosynthetic pathway for nootkatone.
Figure 2 illustrates a Probit analysis survival plot of sea lice against nootkatone dose. The plot shows the range of percentile estimates for EC (effective concentration) values for males, females and total population, respectively, against nootkatone dose.
Figure 3 illustrates a probability plot for a bioassay indicating the LC₅₀ of sea lice using nootkatone.
Figure 4 is GC-FID chromatogram overlay of Frutarom® nootkatone (i.e., citrus-derived nootkatone) and the nootkatone (NxV) used for the treatments described herein (see Examples below).
Figure 5 is a GC-MS NIST library match of an unknown peak in Frutarom® nootkatone. The peak was identified as limonene. Neither limonene nor bergapten was found in the nootkatone used in the present application.

### DETAILED DESCRIPTION

Before describing the present invention in detail, a number of terms will be defined. As used herein, the singular forms "a," "an," and "the" include plural referents unless the context clearly dictates otherwise. For example, reference to "an active ingredient" means one or more active ingredients.

As used herein, the term "active ingredient" refers to a chemical compound or mixture of chemical compounds that kills and/or repels a crustacean ectoparasite (such as a fish louse or sea louse) from at least one of a volume of water and a surface, such as the scales, gills, and/or skin of a fish.

As used herein, the term "crustacean ectoparasite" and "ectoparasitic crustaceans" are used interchangeably, and may refer to a single and/or two or more crustacean ectoparasites of the same or different species. Crustacean ectoparasites of particular economic importance to fish (such as fish bred for food, sport or ornamental value)

As used herein, the term "sea louse" refers to a single sea louse and/or two or more sea lice of the same or different species. As used herein, all sea lice are ectoparasite copepods within the order *Siphonostomatoida.* Optionally, the sea louse is an ectoparasite from one of the following families: *Anthessiidae, Bomolochidae, Caligidae, Cecropidae, Chondracanthidae, Dichelesthiidae, Dissonidae, Ergasilidae, Eudactylinidae, Hatschekiidae, Hyponeoidae, Lernaeopodidae, Lernaeosoleidae, Lernanthropidae, Macrochironidae, Pandaridae, Pennellidae, Philichthyidae, Pseudocycnidae, Sphyriidae, Shiinoidae, Taeniacanthidae, Tegobomolochidae, Telsidae, Tisbidae, Tuccidae.* Optionally, the sea louse is an ectoparasite in the genus *Lepeophtheirus* or *Caligus.* Optionally, the sea louse is *Lepeophtheirus salmonis. Caligus curtus, Caligus musaicus, Caligus vespa, Caligus atromaculatus, Caligus elongatus, Caligus clemensi, Caligus cookeoli, Caligus bonito, Caligus coryphaeae, Caligus inopinatus or Caligus rogercresseyi*.In some aspects the sea louse is a Copepod. In some aspects the sea louse is a *Lepeophtheirus.* In some aspects the sea louse is a *Lepeophtheirus.*

As used herein, the term "fish louse" refers to a single fish louse and/or two or more fish lice of the same or different species. In some aspects the fish louse is a Branchiura. Optionally, the fish louse is of the family *Argulidae.* Optionally, the fish louse is a member of the genus *Argulus, Chonopeltis, Dolops* or *Dipteropeltis.* Optionally, the fish louse selected from at least one of *Argulus foliaceus, Argulus japanicus, Argulus coregoni, Argulus canadnesis, Argulus siamensis, Argulus bengalensis, or Dolops ranarum.*

It is noted that terms like "preferably," "commonly," and "typically" are not utilized herein to limit the scope of the claimed invention or to imply that certain features are critical, essential, or even important to the structure or function of the claimed invention. Rather, these terms are merely intended to highlight alternative or additional features that can or cannot be utilized in a particular embodiment of the present invention.

For the purposes of describing and defining the present invention it is noted that the term "substantially" is utilized herein to represent the inherent degree of uncertainty that can be attributed to any quantitative comparison, value, measurement, or other representation. The term "substantially" is also utilized herein to represent the degree by which a quantitative representation can vary from a stated reference without resulting in a change in the basic function of the subject matter at issue.

As used herein, the term "about" refers to ±10% of any particular value.

As used herein, the terms "or" and "and/or" are utilized to describe multiple components in combination or exclusive of one another. For example, "x, y, and/or z" can refer to "x" alone, "y" alone, "z" alone, "x, y, and z," "(x and y) or z," "x or (y and z)," or "x or y or z."

As used herein, the term "treatment of crustacean ectoparasite" refers to a process by which at least one crustacean ectoparasite is at least one of killed, removed, or repelled from a host surface, such as skin, gills, scales, or other animal surface or other man-made or natural surfaces in the proximity to a treatment population. The crustacean ectoparasite may be treated directly by coming into contact with a contemplated treatment composition or indirectly when fish ingest contemplated treatment compositions included within fish feed.

As used herein, the term "treatment population" refers to one or more animals that may serve as a host to a crustacean ectoparasite of fish.

As used herein, the term "effective concentration" refers to a concentration of an active ingredient (such as nootkatone) within a composition applied to a fish or surface the fish comes into contact with, such that a crustacean ectoparasite coming into contact with said treated fish or treated surface is repelled and/or experiences paralysis, poisoning, neuro-muscular damage, or death.

As used herein, the term "subject" refers to an individual animal, such as a fish.

As used herein, the terms "aquaculture device" or "aquaculture equipment" interchangeably refer to any device and/or apparatus employed in fish farming that either directly or indirectly contacts a fish. Examples of aquaculture devices include, without limitation, boats, nets, floats, tools, buoys, fish cages, tank walls and liners, clothing used when handling fish, such as gloves, boots, coats, waders, etc., aerators, pumps, pipes, breeding chambers, filters, filtration units, incubators, and hatcheries.

As used herein, the terms "fish food" and "feed" are used interchangeably and refer to any food or feed composition provided to fish, such as pellets, chow, kibble, flake, freeze-dried, frozen, and others.

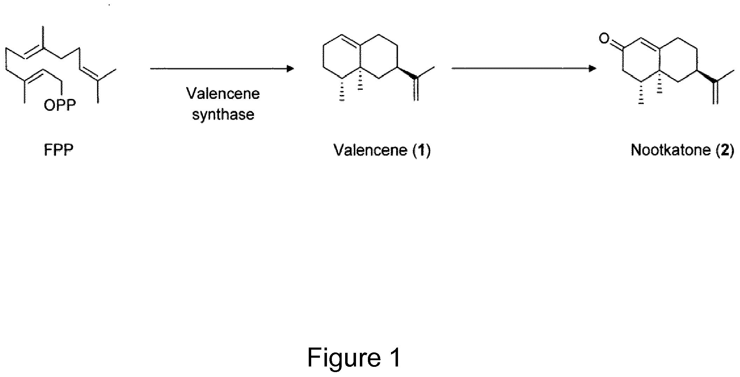

As used herein, the term "nootkatone" refers to a compound seen in Figure 1 that may be synthesized, isolated, and purified from of a mixture of products produced in a host modified to express enzymes of the nootkatone biosynthetic pathway or that can be produced from naturally occurring sources, such as citrus plants. "Nootkatone" also refers to a mixture of chemical compounds containing or enriched for the nootkatone compound and derived from a modified host, such as a microorganism, or isolated or derived from plant extracts.
For example, the nootkatone compound contemplated for use
herein may be produced *in vivo* through expression of one or more enzymes involved in the nootkatone biosynthetic pathway in a recombinant yeast or *in vitro* using isolated, purified enzymes involved in the nootkatone biosynthetic pathway, such as those described in U.S. Patent Application Publication Nos. 2015/0007368 and 2012/0246767.

As used herein, the term "nootkatone ex valencene" refers to nootkatone derived from oxidation of valencene that was produced by fermentation, such as by microorganisms harboring one or more valencene synthases and/or other molecules that catalyze formation of valencene. Further, nootkatone ex valencene refers to a combination of chemical compounds derived from oxidation of a valencene-containing fermentation product produced by culturing microorganisms harboring one or more valencene synthases and/or other molecules that catalyze formation of valencene. Nootkatone ex valencene can be purified to maximize the percent of nootkatone relative to other chemical compounds. For example, nootkatone ex valencene can be less than about 50%, about 50%, about 60%, about 70%, about 80%, about 90%, or about 98% nootkatone.

### OVERVIEW

Disclosed herein are nootkatone-containing compositions and methods of using the compositions that are effective at treating and preventing crustacean ectoparasite infections of fish species in aquaculture facilities and in the surrounding waters of such facilities.

It is one object of some embodiments of the current invention to provide compositions containing nootkatone for use in methods for reducing transfer of crustacean ectoparasite between farmed fish species and wild fish species swimming in the same waters. The present treatments can reduce bio-pollution attributed, in part, to fish farms by reducing spread of crustacean ectoparasites from areas of intensive fish farming to surrounding wild fish populations. In turn, it is believed that fish farm productivity and the quality of harvested fish will be improved by the resultant diminished levels of crustacean ectoparasitesinfection in wild fish populations. In other words, it is believed that treatment of aquaculture facilities according to the present invention will reduce rates of cross contamination of crustacean ectoparasitesbetween farm fish and wild fish populations and thereby improve the health of both populations.

It is another objective of some embodiments of the current invention to provide compositions using nootkatone for use in methods to improve the marketability of farmed fish by reducing the number of crustacean ectoparasite lesions and therefore reduce the number of fish being discarded or not bought by consumers. The greater marketability of farmed fish will improve fish farm efficiency and sustainability.

The compositions for use in treating an infection of an ectoparasitic crustacean on a fish may increase growth rates, decrease mortality rates, and increase resistance to bacterial and viral infections of farmed fish by reducing crustacean ectoparasite infection and thereby improving the effectiveness of the host immune response to sea louse infection. Accordingly, native wild fish populations susceptible to crustacean ectoparasite infection can have improved growth rates, decreased mortality rates, and increased resistance to bacterial and viral infections in areas surrounding fish farms due to treatment of the farmed fish.

The compositions for use in treating an infection of an ectoparasitic crustacean on a fish may directly or indirectly reduce the occurrence or severity of diseases in fish by reducing the prevalence
of crustacean ectoparasite (such as fish lice or sea lice) infections that lead to or exacerbate such diseases. Examples of such diseases include salmon anemia virus, furunculosis, vibriosis, bacterial kidney disease, bacterial gill disease, yersiniosis, white spot, costiasis, ciliated protozoan parasite, kudoasis, and others.

Various methods may be employed to contact fish with nootkatone-containing compositions, such methods including addition of nootkatone-containing compositions to water in which the fish are present, or to water in tanks into which the fish to be treated are introduced, or into fish feed suitable for feeding the fish to be treated, or in compositions rubbed, wiped, brushed, or sprayed onto the fish to be treated, or coated onto a surface of an aquaculture device or impregnated into materials used for aquaculture devices.

Fish to be treated for crustacean ectoparasite can be any fish in need thereof, including farmed fish (i.e., those grown for market) or commensalist or mutualist species cohabiting with farmed fish and/or bred by humans and introduced into fish farms, such as cleaner fish, used to support the farmed fish. In one aspect of the current invention, a population of cleaner fish (such as a wrasse or lumpsucker species) is isolated or grown, treated with a nootkatone-comprising composition (either by surface contact such as in a bath, or by ingestion such as in feed), and then introduced into a fish farm enclosure.

### COMPOSITIONS

Nootkatone-containing compositions contemplated herein can be formulated for direct application topically to a subject in need thereof to treat or prevent infection (as a prophylactic) or by feeding the subject the nootkatone-containing composition. In addition, nootkatone-containing compositions contemplated herein can be formulated for indirect application, such as by dispensing into or onto a zone or area of water in which the subjects are housed. A further manner of indirect application includes coating/treating aquaculture device surfaces or impregnating such aquaculture devices with nootkatone-containing compositions.

Generally and without limitation, compositions contemplated herein can be in the form of an aqueous liquid, an oil-based liquid, a concentrated liquid, a gel, a foam, an emulsion, a slurry, a paint, a clear coat, a wax, a block, a pellet, a puck, a granule, a powder, a capsule, a vesicle, an effervescent tablet, slow release tablet, an impregnated dissolvable sheet or film, an impregnated porous material, an extruded kibble or chow, fishmeal, and combinations thereof.

In certain embodiments, a composition may be formulated for application topically on an exterior surface of a fish, for example, to the skin, gills, eyes, mouth, scales, or fins. In this context, the composition can be provided as an aerosol, a solution, an emulsion, an oil, a lotion, a soap, a spray, a gel, a powder, a foam, and combinations thereof. Similarly, such topical compositions may be applied to surfaces of aquaculture devices.

In certain embodiments, a composition may be formulated for application indirectly by dispensing into or onto a zone of area of water or onto an aquaculture device surface. In this context, the composition can be provided as an aerosol, a solution, an emulsion, an oil, a spray, a gel, a powder, a foam, a paint, a clear coat, a wax, a block, a pellet, a puck, a granule, a vesicle, a powder, a capsule, an impregnated dissolvable sheet or film, and combinations thereof.

In some embodiments, a composition may be formulated as a fish food. In this context, the fish food composition can be either wet or dry, extruded chow, pelletized, wet coated, dry coated, cereal- based, meat-based, and combinations thereof. Nootkatone, additional active ingredients, and/or immuno-stimulating compounds may be added to fish feed compositions either wet or dry, either before or after pelletization, either within the fish food composition or on the surface.

A contemplated fish food composition can include farm waste and/or food industry waste. Typically, the fish food composition can include one or more feed materials or processed derivatives thereof (additives) in any combination selected from: a) cereals, such as small grains (e.g., wheat, barley, rye, oats, and combinations thereof) and/or large grains such as maize and/or sorghum; b) byproducts from cereals, such as corn gluten meal, Distillers Dried Grain Solubles (DDGS) (particularly corn-based Distillers Dried Grain Solubles (cDDGS), wheat bran, wheat middlings, wheat shorts, rice bran, rice hulls, oat hulls, palm kernel, and/or citrus pulp; c) protein or crude protein obtained from sources such as soya, sunflower, peanut, lupin, peas, fava beans, cotton, canola, fish meal, dried plasma protein, meat and/or bone meal, feather meal, poultry meal, fish meal, potato protein, whey, copra, and/or sesame; d) oils and fats obtained from vegetable and animal sources such as rapeseed oil, poultry fat, and/or fish oil; e) minerals such as manganese sulfate, zinc methionine, calcium iodate, copper sulfate, and/or sodium selenite; and f) antioxidants or vitamins such as vitamin E, riboflavin, vitamin B6, vitamin B12, folic acid, vitamin K, and/or ethoxyquin.

Optionally, the fish feed includes at least about 30% by weight crude protein, at least about 30% by weight crude fat or oil, and between about 0.1 and about 10% by weight nootkatone.

Compositions contemplated herein can contain a carrier and at least about 0.1%, or at least about 1%, or at least about 2%, or at least about 5%, or at least about 7.5%, or at least about 10%, or greater than about 10%, or greater than about 15%, or greater than about 20%, or greater than about 25%, or greater than about 50% by weight nootkatone. In some applications, nootkatone can be present in an amount that is greater than about 60%, about 70%, about 80%, about 90%, about 95% or about 99% by weight of the composition. In one example, the provided compositions contain nootkatone in an amount at or about 0.001% to at or about 2%, or about 0.01% to at or about 5%, or about 0.01% to at or about 75% by weight of the composition. In another example, a composition may contain nootkatone in an amount of from at or about 1% to at or about 50% by weight of the composition. In another example, a composition may contain nootkatone in an amount of from at or about 5% to at or about 40% by weight of the composition. In another example, a composition may contain nootkatone in an amount of from at or about 10% to at or about 30% by weight of the composition. In another example, a composition may contain nootkatone in an amount of from at or about 15% to at or about 25% by weight of the composition. In another example, a composition may contain nootkatone in an amount of from at or about 1% to at or about 90% by weight of the composition. In another example, a composition may contain nootkatone in an amount of about 10%, or about 15%, or about 20%, or about 25%, or about 30%, or about 50% by weight of the composition. In another example, a composition may contain nootkatone in an amount of up to about 99% or more by weight of the composition.

A contemplated composition may be seen in Table No. 1.

**Table No. 1. Contemplated composition formulation.**

| **Ingredient** | **Approximate Wt. %** |
|---|---|
| Nootkatone | 0.01 - 100 |
| Additional active ingredients | 0 - 99.9 |
| Carrier | 0 - 99.9 |
| Additives | 0 - 99.9 |

In certain embodiments, compositions contemplated herein may include nootkatone and one or more additional active ingredients. For example, a fish food composition including nootkatone may additionally contain at least one additional active agent selected from an avermectin (such as emamectin benzoate) and teflubenzuron. These drugs have high selective toxicity for sea lice, are lipid-soluble so that there is typically sufficient drug to act for approximately 2 months, and any unmetabolized drug is excreted so slowly that there are little to no environmental concerns. Further, organophosphates and pyrethroids may be added to topical compositions.

Combination of nootkatone with an avermectin can reduce the amount of avermectin needed to be effective and can therefore decrease the rate at which resistance of crustacean ectoparasite such as sea lice to avermectins develops. Similarly, combination of nootkatone with an organophosphate and/or a pyrethroid in a topical formulation can reduce the amount of pyrethroid needed to be effective and can therefore decrease the rate at which resistance of crustacean ectoparasite such as sea lice to pyrethroids develops.

Another additional active ingredient in the context of treat aquaculture equipment includes peroxide.

Compositions contemplated herein may include nootkatone in combination with one or more additive, such as a fragrance, a preservative, a propellant, a pH buffering agent, a *uv* blocker, a pigment, a dye, a surfactant, an emulsifier, a solvent, a salt, an antibiotic, an analgesic, a binder, a filler, an acid, a base, an emollient, a pharmaceutical excipient, and combinations thereof. Additional additives can be selected from among agents that are capable of stimulating the immune system, such as resveratrol, that are capable acting as a CYP P450 agonist, and agents that are capable of acting as an analgesic, such as the prostaglandin E2 inhibitors of as aspirin, ibuprofen, or other nonsteroidal anti-inflammatory drugs (NSAIDS). An additive may be added to a composition in an amount of about 1% to about 50%, or about 5%, or about 10%, or about 15%, or about 20%, or about 25%, or about 30%, or about 50% by weight of the composition.

Compositions may include a carrier, such as an aqueous liquid carrier, water, a saline, a gel, an inert powder, a zeolite, a cellulosic material, a microcapsule, an alcohol such as ethanol, a hydrocarbon, a polymer, a wax, a fat, an oil, and the like. A carrier may be added to a composition in an amount of about 10%, or about 15%, or about 20%, or about 25%, or about 30%, or about 50% by weight of the composition. In some applications, a carrier can be present in an amount that is at or greater than about 60%, about 70%, about 80%, about 90%, about 95%, or about 99% by weight of the composition.

### METHODS

Nootkatone-containing compositions may be applied to fish infected by crustacean ectoparasite (such as fish lice or sea lice) at any stage of the crustacean ectoparasite life cycle after the egg stage. The treatment of fish with nootkatone may be routine, prophylactic, preventative based on changed environmental conditions (such as water temperature), seasonal, or in response to the detection of an elevated incidence of crustacean ectoparasite in the fish farm population, the populations of adjacent fish farms, or the wild population of a native fish species. Treatment of fish with compositions comprising nootkatone can be initiated for at least part of a fish farm if there is observed a per-fish average of more than 0.5 sexually mature female lice or three mobile lice per fish, and the entire fish farm receives treatment against crustacean ectoparasites within 14 days. In another embodiment, a coordinated preventative treatment is performed in a fish farm during periods of seasonal migration of wild smolt and/or adult fish through the farm.

Topical treatment of infected fish can be accomplished by netting infected fish and applying a contemplated composition by hand (brushing, spraying, sponging, dipping, etc.). Alternatively, the infected fish may be placed in a separate or isolated tank or "well boat" for a "bath treatment" where a nootkatone-containing formulation is added to the well. Use of well boats can reduce the amount of composition required, reduce some environmental concerns, and treat fish in a more uniform manner. A non-limiting example of a typical treatment volume in a well boat may be about 200 m³ to about 500 m³, or between about 300 m³ and about 400 m³.

Alternatively, infected fish populations may be treated *in situ* within their tanks, cages, or nets. Fish farming tanks, nets, and cages used within the industry include floating cages, closed sea-going units, submersible offshore farming cages, closed containment cages, and converted bulk ships. If the fish containment is nets or cages, they can be at least partially isolated from the surrounding water using such water-impermeable barriers as tarpaulin and/or skirts. Enclosure within a full tarpaulin envelope is preferred to partial enclosure (such as with skirts). Preferably, treatment of such fully contained fish with compositions comprising nootkatone is performed using countercurrent. Preferably, the oxygen content of the treatment water is monitored either continuously or at least once every 10 minutes, and the treatment water is oxygenated during fully enclosed treatment to maintain fish health and reduce stress.

Fish containment tanks can be subdivided by inserting impervious dividing walls made of Plexiglas® or a canvas-type material into their wells to divide the treatment space into separate "baths" or treatment areas. Further skirts or tarpaulins can be placed within or around the cages to at least partially contain the applied composition. For example, a skirt can surround a fish cake or net, and a tarpaulin can be suspended at a depth of between about 4 m and about 5 m within a typical commercial fish farming cage of about 900 m², providing a treatment volume of approximately 3,600 m³. Optionally, the host fish can be starved for about 2 days prior to reduction of containment volume or introduction into a smaller volume net, tank, cage or bath for treatment with nootkatone comprising compositions. Compositions comprising nootkatone may be applied to the treatment volume at suitable dilutions to provide a treatment concentration of between about 0.01 g to about 1 g nootkatone per m³.

Nootkatone can be applied, such as by directly sprinkling or pouring the composition into the water or placing a composition dispenser within the well, bath, or tank such that the fish to be treated come into contact with the nootkatone at an effective concentration of, for example, between about 100 and about 2000 ppm, preferably between about 200 and about 400 ppm, most preferably approximately 300 ppm. The fish may be exposed to any of the contemplated nootkatone-including compositions for about 15 minutes to about 24 hours. In a preferred embodiment of one aspect of the current invention, the fish are exposed to an effective amount of nootkatone for killing or preventing infection of crustacean ectoparasite, such as, at a concentration of about 300 ppm, for between about 15 to about 60 minutes. In the case of treating fish within their growth cages, nets, or tanks, the timing of exposure to nootkatone begins when all of the composition comprising nootkatone has been added to the treatment volume. Optionally, the fish can be treated until such time as at least one crustacean ectoparasite is seen to detach or become immobile.

The nootkatone-comprising composition can be applied to the fish or surface at a concentration sufficient to cause at least about 50% of the female crustacean ectoparasite population (more preferably at least about 50% of the entire crustacean ectoparasite population) to become weak and/or moribund. Optionally, the nootkatone-comprising composition can be applied to the fish or surface at a concentration of between about 4 and about 50 mg/L, such as between about 4 and about 12 mg/L. Weak and/or moribund crustacean ectoparasite are more likely to drop off or be brushed off host fish and surfaces (such as fish farm apparatus). In some instances, crustacean ectoparasites that are weak or moribund following contacting of host fish with compositions comprising nootkatone can be removed from the host fish by mechanical crustacean ectoparasitesremovers, such as by permitting the fish to squeeze through flexible large nets, said nets can be optionally coated or impregnated with compositions comprising nootkatone. In further instances, such treatment with nootkatone can optionally be followed by physical crushing or laser treatment of the weak or moribund crustacean ectoparasitesat one surface (preferably the bottom) of the container into which the nootkatone-comprising composition was administered (such as the treatment container on a well boat or isolated treatment tank).

In contrast to many pesticides in the art for fish treatment, nootkatone is able in some compositions of the current invention to form a film on the surface of water. Therefore, some methods employ procedures of transferring the fish into nootkatone-containing baths or tanks such that the fish to be treated must pass through the surface of the water such that a film of nootkatone at least partially envelopes the fish for a period of time. For example, new fish introduced into a well or tank may be first dipped into a composition containing nootkatone and then placed in the well or tank. Alternatively, a fish to be introduced to a well or tank may pass through chute or pipe inserted into the well or tank that is filled with the nootkatone-containing composition and thus requires the fish to swim through the composition to enter the well or tank.

Larger bath volumes may be utilized to reduce fish stress due to crowding. In this context, longer treatment times in the compositions of the current invention including nootkatone are contemplated to compensate for potentially lower concentrations of nootkatone. Optionally, fish are placed in contact with nootkatone at a concentration of at least about 800 ppm for at least about 1 day. In other options, fish may be placed in contact with nootkatone at a concentration of at least about 100 ppm for at least about 5 days.

Optionally, fish feed compositions including nootkatone are provided to the fish to be treated in late spring. Additionally or alternatively, fish feed compositions including nootkatone may be provided to the fish to be treated every 17 to 72 days depending on water temperature.

In a preferred option, nootkatone is added to a closed containment system, such as a tank or isolated enclosure populated or due for population by juvenile fishes. For example, smolts (young fish of between 100 and 150 g) can be treated with compositions comprising nootkatone for between about 1 and 7 days in small volume tanks just before transfer to seawater. In another option, net-cage aquaculture systems may be treated. Closed containment systems can offer the advantage of recycling the nootkatone-containing composition multiple times through a circular flow system. It is further envisioned that such closed containment systems may incorporate filters to catch detached crustacean ectoparasitesas they release from the treated fish due to treatment with nootkatone-containing compositions and permit a user to remove the crustacean ectoparasitesfrom the water.

### DISPENSERS

Topical compositions disclosed herein can be dispensed using one or more of a perforated hose, a spray bottle, a brush, a dropper, a sponge, a soft-tipped marking device with reservoir, a pressurized dispenser, an aerosol can, a wipe, and other devices suitable for topical application.

It is also disclosed that, methods for treating fish can include utilization of nootkatone-containing composition dispensers that release a contemplated composition into a body of water (treatment locale) over a period of time of minutes, hours, days, or weeks. Contemplated dispensers include floating dispensers that float and dispense at the surface of a body of water. For example, contemplated floating dispensers include those that are similar to or the same as are used for chlorine dispensing in swimming pools. Floating dispensers can float on the surface of a locale either freely or can be anchored. Further, when anchored, floating dispensers can float subsurface at a predetermined and adjustable depth. In a preferred aspect, a floating dispenser, such as a perforated hose, is moved around a treatment locale (such as an enclosed reduced volume fish tank, net, or cage) during the period of administration of the composition comprising nootkatone.

Other dispensers include sink-floats that can be immersed within a treatment area and sink to or near the bottom of a treatment locale until such time as the treatment composition is completely released, at which time the dispenser floats to the surface to be recharged. In another embodiment, a weighted and buoyed dispenser can be used that includes a weighted composition dispenser connected to a floating buoy that suspends the dispenser at a predetermined depth at which depth the composition is dispensed.

It is contemplated that a "use up cue" can be included in the contemplated dispensers, such as, for example, a beacon that gives off light and/or sound or changes color when a treatment composition has been nearly or completely used up. The use up cue can be based on a timer, in that, after a predetermined length of time that coincides with the time when the treatment composition is nearly or fully dispensed, the use up cue is triggered by the timer.

In the context of the sink float, the use up cue can function based on the rate of solubility of the treatment composition, such that when the treatment composition is fully dissolved, the weight loss from the sink float causes the float to rise to the surface. Alternatively, the use up cue of the sink float can be based on the rate of solubility of a companion substance within the sink float that dissolves at a rate corresponding to the rate of dispensing of the treatment composition.

Once infected fish have been treated, it is important to try to reduce reintroduction of crustacean ectoparasitess. Therefore, contaminated aquaculture equipment should be treated to remove any crustacean ectoparasitessthat may have attached to surfaces of the equipment. Treatment of such equipment can be accomplished in similar manners as are employed for topical application of nootkatone-containing compositions directly to fish, as described herein elsewhere. For example, aquaculture equipment can be sprayed, brushed, wiped, dipped, and/or soaked with a nootkatone-containing composition.

Also disclosed is the pretreatment of aquaculture equipment to deter attachment of crustacean ectoparasites to a surface thereof. This can be accomplished by coating the equipment with compositions that resist removal from the surface and contain an amount of a nootkatone, such as a paint, a clear coat, a wax, an oil, an adhesive, a resin, and combinations thereof. Another approach includes lining the aquaculture equipment with one or more nootkatone-impregnated materials, such as thermoplastic or thermoset sheets impregnated with nootkatone. A further approach is to construct the aquaculture equipment of nootkatone impregnated materials, such as plastics, wood, cloth, textiles, composites, porous materials to prevent re-infection of fish between treatments and/or between harvests. These approaches can be used alone or in any combination.

### FEEDING

In-feed treatments are easier to administer to the entire farmed fish population within a locale. However, an acknowledged disadvantage of in-feed treatments is that the amount of drug ingested can vary from fish to fish. Due to the nature of nootkatone, it is possible to place a requisite dose in every feed pellet or other unit of food to ensure sufficient dosage to every feeding fish, yet no detrimental effects will result if a single fish eats more than one pellet.

Nootkatone-containing compositions can be susceptible to oxidation due to exposure to sunlight. Therefore, it is further envisioned to treat fish with nootkatone-containing compositions in shaded and/or covered or otherwise areas that are protected from sunlight of an intensity to reduce the effectiveness of the nootkatone-containing composition. For example, the nootkatone-containing composition can be administered to a population of fish in a covered tank, a covered net, a live well, and/or the well of a well boat.

The invention will be further described in the following examples, which do not limit the scope of the invention described in the claims. Any examples which fall outside of the scope of protection provided by the appended claims are provided for reference only.

### EXAMPLES

The Examples that follow are illustrative of specific embodiments of the invention, and various uses thereof. They are set forth for explanatory purposes only and are not taken as limiting the invention.

### Example No. 1: Susceptibility of sea lice to treatment with nootkatone formulations.

This example describes a laboratory bioassay in which groups of sea lice were exposed to solutions of nootkatone at different concentrations to determine sea lice susceptibility to nootkatone.

Tests were conducted using *Lepeophtheirus salmonis* sea lice produced from laboratory maintained cultures collected from Atlantic salmon maintained in 3 m diameter (7 m³) GRP seawater tanks. Fish were anaesthetized (MS222 at 100 mg/L) and pre-adult II and/or adult lice were removed using forceps, placed into 100 micron filtered seawater in polythene bags, and then transferred to an incubator at a temperature of 12.0°C.

Each bioassay was performed using similar numbers of male and female pre-adult II and/or adult sea lice. Groups of approximately 10 sea lice (preferably 5 male and 5 female) were randomly allocated into three replicates per concentration and exposed to each test concentration mixed with seawater. During allocation, any lice which seemed abnormal were replaced before the start of treatment.

The exposure solutions were prepared using the stock test material formulations, and seawater as follows. One gram of Nootkatone 98% w/w was dissolved in 1 mL of absolute ethanol (technical grade) to form the stock test material formulation (Solution 1). 1 mL of Solution 1 was then added to 999 mL seawater filtered through a 100 µm filter to give Solution 2 (at 980 mg nootkatone per L seawater). Various volumes of Solution 2 were further diluted in filtered seawater as described in Table No. 2 to provide final test volumes of 250 mL of solutions for bioassay exposure giving a circa 242 fold difference in concentration between the lowest and highest test concentrations. Seven test concentrations (0.04-980 mg/L) plus two seawater controls (0 mg/L) were used in each bioassay. Solutions were prepared within 2 hours of the start of the assay.

**Table No. 2. Experimental treatment groups and exposure concentrations (mg/L) for nootkatone bioassay tests on sea lice.**

| **Treatment** | **Dose ppm** | **Vol. of Solution 2 (mL)** | **Vol. of seawater (mL)** | **Final test Volume (mL)** |
|---|---|---|---|---|
| 1 | 0 | 0.00 | 250.00 | 250.00 |
| 2 | 4.03 | 1.03 | 248.97 | 250.00 |
| 3 | 12.10 | 3.09 | 246.91 | 250.00 |
| 4 | 36.30 | 9.26 | 240.74 | 250.00 |
| 5 | 108.89 | 27.78 | 222.22 | 250.00 |
| 6 | 326.67 | 83.33 | 166.67 | 250.00 |
| 7 | 980.00 | 250.00 | 0.00 | 250.00 |

A total of 250 mL of each exposure solution were made and then placed in the LMS incubator and allowed to acclimate to a temperature of 12.0 ± 1.0°C. Sea lice were added to Petri dishes containing 70 mL of each test solution. Clean glassware was used for each assay. Groups of 10 sea lice in three replicates were exposed to each test concentration and seawater control, respectively. Tests were performed using similar numbers of male and female pre-adult II/adult sea lice.

All groups were exposed to test concentrations for 24 hours. At the end of the exposure period, sea lice mortality and morbidity were assessed using an Olympus SZ30 microscope. Sea lice exposed to the lowest treatment dose of test concentration were examined first, thereafter, subsequent doses in increasing order. The viability of each individual louse was determined at 24 hour post exposure and scored as alive, weak, moribund, or dead. Viability was classified (A-D) as follows: (A) alive/unaffected (attached and/or actively mobile); (B) weak (unattached, unusual mobility); (C) moribund (unattached, immobile but movement of appendages); and (D) dead (immobile and no movement of appendages). For adult lice, data for males and females were recorded separately.

The proportion of dead and moribund plus dead sea lice was determined at each test product concentration and replicates averaged. The 24 hour EC₅₀, EC₉₀, LC₅₀, and LC₉₀ values were calculated using probit regression analysis. EC₅₀ (effective concentration 50%) is defined as the concentration at which 50% of lice are either moribund or dead when assessed at 24 hour post exposure, LC₉₀ (lethal concentration 90%) is the concentration at which 90% of lice are dead when assessed at 24 hour post exposure. Percentage efficacy was calculated for each test concentration relative to the controls at each dose. The data were used to establish survival curves and establish effective concentrations (EC₅₀, EC₉₀) and lethal concentrations (LC₅₀, LC₉₀).

### Results

As shown in Table No. 3, microscopic observations of sea lice at 24 hours post-exposure revealed an inverse relationship between viability and nootkatone dose. There were no viable sea lice observed at concentrations ≥ 38 mg/L. Controls displayed > 80% viability indicating good health of the test parasites and the validity of the bioassay.

As shown in Table No. 4, EC₅₀ values were 11.54 mg/L [95% Cl 7.45-17.17 mg/L] for male sea lice and 9.17 mg/L [95% CI 7.05-11.41 mg/L] for female sea lice. EC₉₀ values were 24.26 mg/L [95% CI 18.27-38.55 mg/L] for male sea lice and 13.43 mg/L [95% CI 11.23-18.18 mg/L] for female sea lice. LC₅₀ values were 16.39 mg/L [95% CI 11.78-23.17 mg/L] for male sea lice, while lower values were obtained for females at 9.69 mg/L [95% CI 7.53-12.23 mg/L].

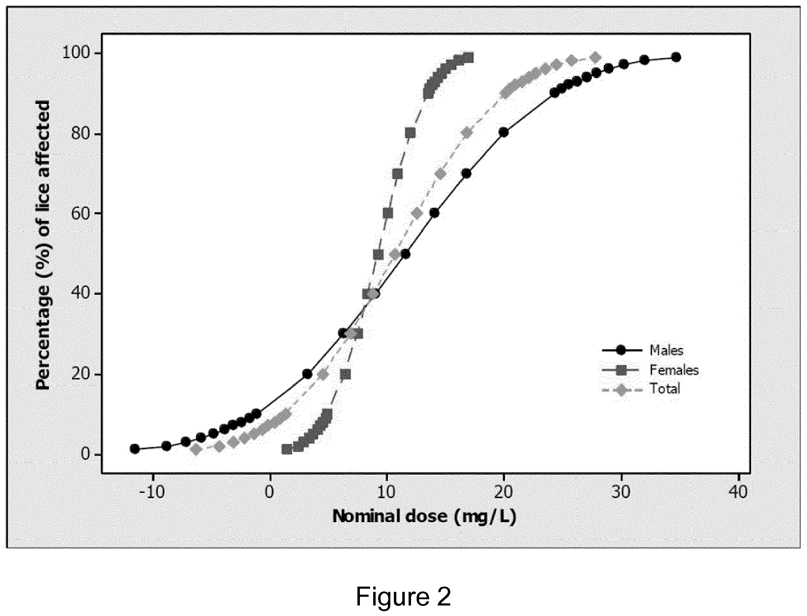

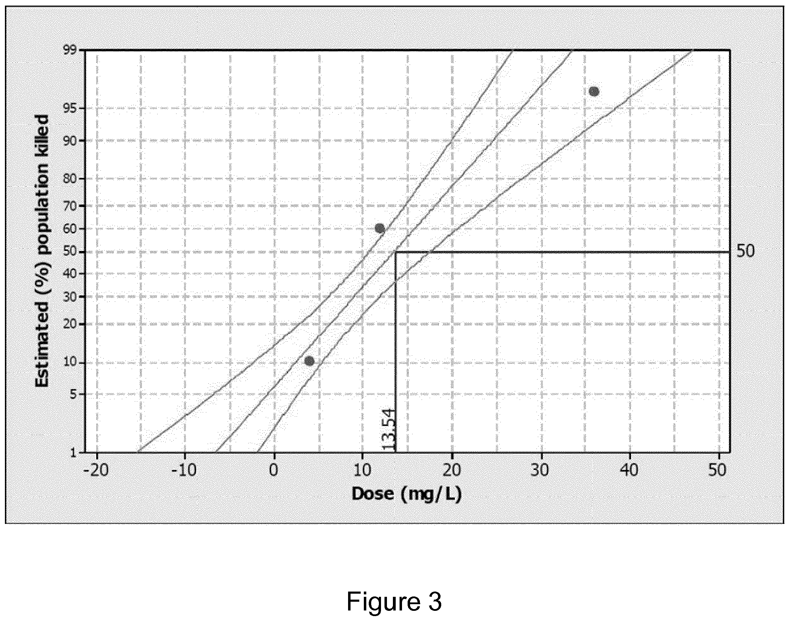

The full range of percentile estimates for EC values of nootkatone calculated by probit analysis are plotted as shown in Figure 2. Sea lice males appear to be more resistant than females. The estimates generated by Minitab for LC₅₀ and LC₉₀ are displayed using a probability plot for the bioassay data, which indicate that a dose of 13.54 mg/L would kill 50% of the total population (Figure 3).

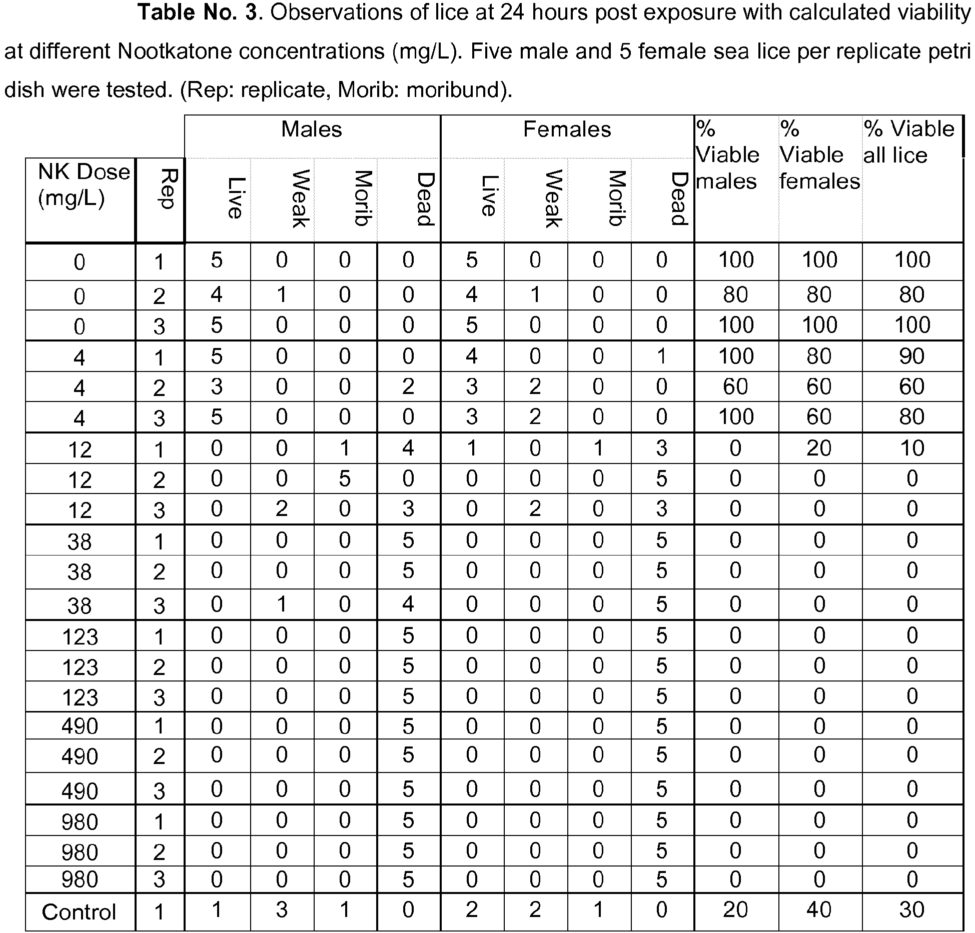

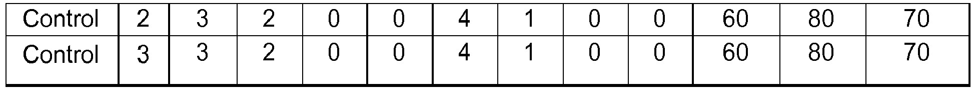

**Table No. 4. EC₅₀ values for adult male and adult female (pre-ovigerous) L. salmonis at 24 hours post exposure to nootkatone. All values are mg/L, with 95% confidence intervals reported. LW 95%: lower 95% confidence interval, UP 95%: upper 95% confidence interval.**

| **Males EC₅₀** | **LW 95%** | **UP 95%** | **Females EC₅₀** | **LW 95%** | **UP 95%** |
|---|---|---|---|---|---|
| 11.54 | 7.45 | 17.17 | 9.17 | 7.05 | 11.41 |

| **Males EC₉₀** | **LW 95%** | **UP 95%** | **Females EC₉₀** | **LW 95%** | **UP 95%** |
|---|---|---|---|---|---|
| 24.27 | 18.27 | 38.56 | 13.43 | 11.23 | 18.19 |

| **Males LC₅₀** | **LW 95%** | **UP 95%** | **Females LC₅₀** | **LW 95%** | **UP 95%** |
|---|---|---|---|---|---|
| 16.40 | 11.79 | 23.18 | 9.70 | 7.53 | 12.23 |

### Discussion

The present study indicated that no viable sea lice were observed after exposure to nootkatone in concentrations of 38 mg/L or higher. Sea lice populations experienced 50% mortality when treated with between 15 mg/L and 20 mg/L, with the average LC₅₀ for the total sea lice population (male and female) being about 13.5 mg/L. Overall, nootkatone displayed high efficacy against sea lice.

Of further note is that female sea lice are more susceptible to nootkatone than male sea lice. This has the unexpected benefit of enabling a lower concentration of nootkatone to be used to prevent or treat a breeding population of sea lice by selectively removing female sea lice from a mixed population. For example, Figure 2 shows that the female sea lice population is 100% affected by nootkatone at a concentration between approximately 15 and 20 mg/L.

### Example No. 2: Comparison of fermentation-derived nootkatone with citrus-derived nootkatone

### Overview

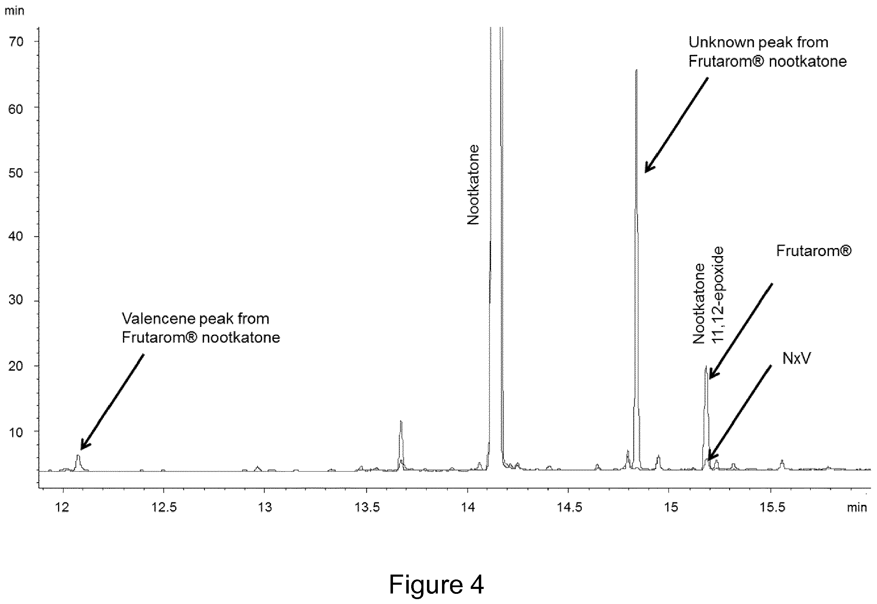

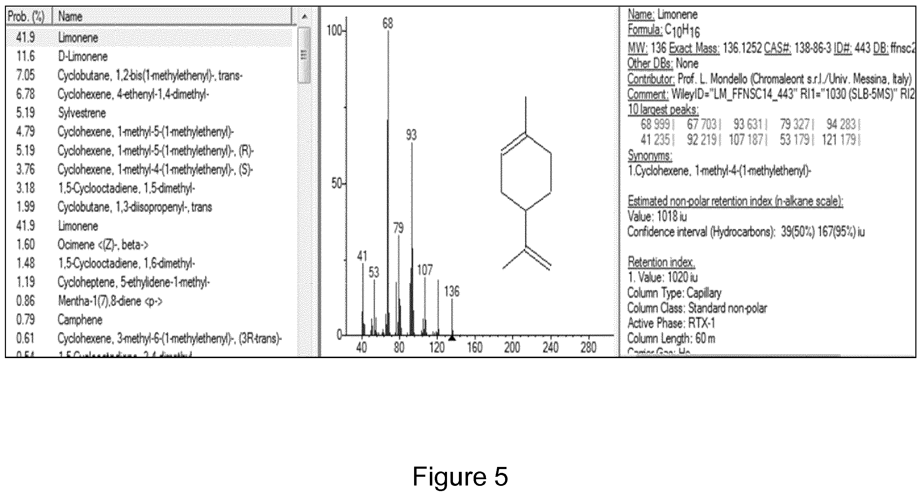

Nootkatone, as defined herein, has a particular chemical profile indicative of its constituent chemical species. Other sources of nootkatone can have different chemical profiles and therefore actually represent different chemical compositions. GC-FID analyses of the nootkatone used in the studies described above (obtained from oxidation of fermentation-derived valencene, also known as, nootkatone ex valencene (NxV)) and a citrus fruit-derived nootkatone (also known as nootkatone ex citrus, which is derived from citrus fruit and available from Frutarom®, Corona, CA) are shown in Figure 4. The nootkatone used in the studies described herein lacked valencene and demonstrated a lower amount of 11,12-epoxide than the Frutarom® nootkatone. Moreover, further analysis of an unknown peak from the Frutarom® nootkatone sample revealed that the Frutarom® sample contained limonene (see Figure 5), whereas the nootkatone used in the present studies was limonene-free. These results underscore the different chemical profile of the nootkatone used herein (NxV) compared to commercially-available nootkatone derived from citrus, such as that provided by Frutarom®.

These results are also in accord with the observation (not shown) that nootkatone obtained from fermentation-derived valencene does not contain bergapten (or bergaptine). Bergapten (5-methoxypsoralen or 5-MOP) is a compound found in cedar, bergamot and citrus essential oils that causes phototoxicity in humans. (Gionfriddo et al. "Elimination of Furocoumarins in Bergamot Peel Oil," Perfumer & Flavorist., 2004; 29:48-52; Ferreira Maia et al. "Plant-based insect repellents: a review of their efficacy, development and testing," Malaria Journal, 2011; 10:Suppl1-1 1; and Kejlová et al. "Phototoxicity of bergamot oil assessed by in vitro techniques in combination with human patch tests." Toxicol In Vitro. 2007; 21:1298-1303). In addition, GHS health warning statements for bergapten indicate that it can cause allergic skin reactions, allergy or asthma symptoms, or breathing difficulties if inhaled, and can cause genetic defects or cancer in animals. For such reasons, a Cosmetic Ingredient Review expert panel in assessing the safety of 14 citrus-derived peel oil ingredients concluded no more than 0.0015% (15 ppm) bergapten should be included in cosmetic products (*see* "Safety Assessment of Citrus-Derived Peel Oils as Used in Cosmetics," Cosmetic Ingredient Review Expert Panel Final Report, September 30, 2014: 1-31).

Bergapten is present in natural sources of valencene and nootkatone (including citrus and cedar). Nootkatone of less than 98% purity derived from *Chamaecyparis nootkatenisi* is considered likely to comprise allergenic contaminants (Ferreira Maia et al. "Plant-based insect repellents: a review of their efficacy, development and testing," Malaria Journal, 2011; 10:Suppl1-11). Bergapten is a phototoxin. Photoactivation of bergapten by exposure to light may lead to a chemically induced irritation of the skin or exposed membranes. Photoirritation, also called phototoxicity, can be worse around an open wound caused by crustacean ectoparasitesor particularly sensitive membranes of the body. Bergapten-associated adverse reactions in the lungs of fisheries workers or fish produce workers repeatedly exposed to photo-activated bergapten could lead to health complications such as irritation or breathing difficulties. Bergapten-associated adverse reactions resulting from ingested fish food contacted with bergapten could lead to intestinal discomfort. Further, ingestion of fish or fish produce comprising photoactivated bergapten could lead to intestinal discomfort for humans or animals who have eaten the fish or fish produce. Of additional relevance to aquaculture efficiency and the impact of the fish farming industry on the surrounding environment, studies performed by Paik et al. ("A Chemical Genetic Screen in Zebrafish for Pathways Interacting with cdx4 in Primitive Hematopoiesis," Zebrafish, 2010_Mar; 7(1): 61-68) showed one third of fish embryos exposed to 100µM bergapten were killed and half of the remaining viable embryos exhibited defects in anterior-posterior patterning.

Bergapten-free nootkatone obtained from fermentation-derived valencene is preferable for treating fish for crustacean ectoparasites infestations, treating aquaculture equipment, or treating other surfaces humans may come into contact with.

### Example No. 3: Production of Nootkatone ex Valencene

Nootkatone *ex* valencene can be produced *in vivo* through expression of one or more enzymes involved in the nootkatone biosynthetic pathway in a recombinant yeast or *in vitro* using isolated, purified enzymes involved in the nootkatone biosynthetic pathway, such as those described in U.S. Patent Application Publication Nos. 2015/0007368 and 2012/0246767. The final conversion of valencene to nootkatone may be done enzymatically *in vivo* or *in vitro,* or may be performed by chemical oxidation (typically inorganic) *in vitro.*

Briefly, the valencene synthase gene (CVS) from *Citrus sinensis* cv. Valencia (Valencia orange) was cloned from RNA isolated from the juice vesicles of freshly harvested Valencia orange using the procedure previously described in Example 1 of U.S. Pat. No. 7,442,785.

First, Yep-GW-URA (Takahashi et al., (2007) Biotechnol Bioeng. 97(1): 170-181) was generated by inserting a gateway cloning cassette (RfB) with the form attR1-Cm^{R}-ccdB gene-attR2 (Hartley et al., (2000) Genome Res. 10:1788-1795) into the Smal restriction site of YEp352-URA (Bio-Technical Resources), which contains an URA3 selectable marker, an ADH1 promoter and an ADH1 terminator flanking, two BamHI sites (one 5' to the ADH1 promoter and the other 3' to the ADH terminator), a 2-micron ori, an ampicillin resistance gene and a colE1 origin of replication. The resulting vector was designated YEp-CVS-URA.

The CVS gene (set forth in SEQ ID NO: 1, and encoding amino acid sequence is set forth in SEQ ID NO: 2) was then amplified from RNA isolated from the juice vesicles of freshly harvested Valencia orange to contain restriction sites for subcloning into the yeast shuttle expression vector Yep-GW-URA. Following digestion of Yep-GW-URA with EcoRI and Xbal, the amplified product was cloned into the yeast shuttle expression vector YEp-GW-URA.

The YEp-CVS-ura vector was maintained in *S. cerevisiae* by selecting on SD minimal medium lacking uracil at 28° C. The vector also was maintained in *Escherichia coli* by selecting for resistance to ampicillin on LB medium containing 100 µg/mL ampicillin.

To screen for production of valencene, the *Saccharomyces cerevisiae* yeast cell strains CALI5-1 (ura3, leu2, his3, trp1, Δerg9::HIS3, HMG2cat/TRP1::rDNA, dpp1, sue), ALX7-95 (ura3, his3, trp1, Δerg9::HIS3, HMG2cat/TRP1::rDNA, dpp1, sue) or ALX11-30 (ura3, trp1, erg9def25, HMG2cat/TRP1::rDNA, dpp1, sue) were used.

The CALI5-1 strain (see U.S. published Appl. No. US20040249219; U.S. Pat. Nos. 6,531,303 and 6,689,593) has a Δleu2 deletion, which required the introduction of leucine into its media. ALX7-95 was derived from CALI5-1 by correcting the Δleu2 deficiency of CALI5-1 with a functional LEU2 gene (see U.S. published Appl. No. US2010/0151519).

ALX11-30 was constructed from CALI5-1 in several steps from ALX7-175.1 as described in US2010/0151519. Briefly, ALX7-95 HPS was obtained by transforming a plasmid containing the *Hyoscyamus muticus* premnaspirodiene synthase (HPS) into ALX7-95 strain. The YEp-HPS plasmid was obtained by cloning the gene for HPS into Yep-GW-URA to give YEp-HPS-ura (YEp-HPS). Then, an error prone PCR reaction of the ERG9 gene was performed, and the resulting DNA was transformed into ALX7-95 harboring YEpHPS. Transformants were plated on YP medium lacking ergosterol and screened for premnaspirodiene production. Those that produced high levels of premnaspirodiene were saved. One strain, ALX7-168.25 [ura3, trp1, his3, erg9^{def}25, HMG2cat/TRP1::rDNA, dpp1, sue, YEpHPS] was transformed with a PCR fragment of the complete HIS3 gene to create a functional HIS3 gene. Transformants were isolated that were able to grow in the absence of histidine in the medium. From this transformation, ALX7-175.1 was isolated [ura3, trp1, erg9def25, HMG2cat/TRP1::rDNA, dpp1, sue YEpHPS]. Finally, the plasmid YEpHPS was removed by growing ALX7-175.1 several generations in YPD (10 g/L yeast extract, 20 g/L peptone, 20 g/L glucose) and plating cells on YPD plates. Colonies were identified that were unable to grow on SD medium without uracil (0.67 Bacto yeast nitrogen base without amino acids, 2% glucose, 0.14% yeast synthetic drop-out medium without uracil). This strain was designated ALX11-30.

For screening for production of valencene by valencene synthase or mutants, the YEp-CVS-ura plasmid, containing the CVS gene or modified versions of the CVS gene, was transformed into the above yeast strains using the lithium acetate yeast transformation kit (Sigma-Aldrich). The ALX7-95 and ALX11-30 strains generally produced more valencene than the CALI5-1 strain. CALI5-1 was used for initial screening in vials (as described in Example 3) and production in fermenters. Subsequently, ALX7-95 or ALX11-30 were used for screening in vials and fermenters. Typically, ALX7-95 was used for screening in vials and ALX11-30 was used for fermenters.

Transformants were selected on SDE-ura medium (0.67% Bacto yeast nitrogen base without amino acids, 2% glucose, 0.14% yeast synthetic drop-out medium supplement without uracil, and 40 mg/L ergosterol as needed). Colonies were picked and screened for valencene production using the microculture assay described below.

Production of valencene was performed in a 3-L fermentation tank (New Brunswick Bioflow 110). One liter of fermentation medium was prepared and autoclaved in the fermentation tank (20 g (NH₄)₂SO₄, 20 g KH₂PO₄, 1 g NaCl, MgSO₄.7H₂O, 4 g Solulys corn steep solids (Roquette)). The following components were then added: 20 ml mineral solution (0.028% FeSO₄.7H₂O, 0.029% ZnSO₄.7H₂O, 0.008% CuSO₄.5H₂O, 0.024% Na₂MoO₄.2H₂O, 0.024% CoCl₂.6H₂O, 0.017% MnSO₄.H₂O, 1 mL HCI); 10 mL 50% glucose; 30 mL vitamin solution (0.001% biotin; 0.012% calcium pantothenate, 0.06% inositol, 0.012% pyridoxine-HCI, 0.012% thiamine-HCI); 10 mL 10% CaCl₂, and 20 mL autoclaved soybean oil (purchased from local groceries). For sterol-requiring strains, including CALI5-1 and ALX7-95, 50 mg/L cholesterol or 40 mg/L ergosterol was included in the medium.

The seed culture for inoculating the fermentation medium was prepared by inoculating 50 mL of SDE-ura-trp medium (see Example 3.C.2.) with CALI5-1, ALX7-95 or ALX11-30 containing the YEp-CVS-ura plasmid. This culture was grown at 28°C. until early stationary phase (24-48 hr). One mL of this culture was inoculated into 500 mL of SDE-ura-trp medium and grown for 24 hr at 28° C. A 50-mL aliquot (5% inoculum) was used to inoculate the medium in the fermentation tank.

The fermentor was maintained at 28°C. The air flow was 1 vvm and the dO₂ was maintained above 30% by adjusting the agitation. The pH was maintained at 4.5 using phosphoric acid and NaOH or NH₄OH.

When the glucose concentration fell below 1 g/L, a feeding regimen was initiated such that the glucose in the fermentor was kept between 0 and 1 g/L. The glucose feed consisted of 60% glucose (w/v).

At the end of the fermentation, generally about 132 hours after inoculation, sodium sulfate was added to 10-15% final concentration as was an additional 50 mL soybean oil, and the contents of the fermentor were agitated for one hour. After allowing the fermentation vessel contents to settle, the oil was recovered by centrifugation and the valencene content in the oil was determined.

To assay valencene, 3 mL of suspension was placed in a vial to which 3 mL of acetone containing 20 mg/L cedrene was added. After vortexing, the mixture was extracted with 6 mL hexane containing 10 mg/L hexadecane followed by additional vortexing. The organic phase was transferred to a second vial for analysis by gas chromatography using cedrene and hexadecane as internal standards for extraction efficiency and injection, respectively. The CALI5-1, ALX7-95 or ALX11-30 S. *cerevisiae* containing Yep-CVS-ura, and expressing valencene synthase, was found to produce valencene.

The valencene-containing soybean oil, produced by fermentation as described above, was concentrated and purified using wiped-film distillation at 100°C. and 350 mTorr to generate an oil that contained approximately 68% valencene by weight. This material was converted to nootkatone by two different methods described below.

### A. Oxidation of Valencene to Nootkatone Using Chromium Trioxide

The valencene distillate produced as described above was oxidized to nootkatone using chromium trioxide and pyridine in dichloromethane as follows. Chromium trioxide (369 g, 3.69 mol, 22 eq) was added in portions to a solution of pyridine (584 g, 7.4 mol, 44 eq) in 5 L of dichloromethane. The mixture was stirred for 10 minutes, 50 grams of valencene distillate (68% w/w, 0.167 mol, 1 eq) was added over four minutes, and the mixture was stirred at 22° C. for 18 hours. The liquor was drained from the vessel, and the solids were washed twice with 2 L of methyl tert-butyl ether (MTBE). The combined organic layers were further diluted with 2 L of MTBE and successively washed three times with 1.25 L of 5% sodium hydroxide, twice with 2 L of 5% hydrochloric acid, and once with 2 L of brine. The organic phase was dried over 200 grams of anhydrous sodium sulfate, filtered, and concentrated by evaporation to give 36.8 grams crude nootkatone (48% w/w, 0.081 mol, 48% yield).

### B. Oxidation of Valencene to Nootkatone Using Silica Phosphonate-Immobilized Chromium (III) Catalyst

Silica phosphonate chromium (III) resin (48.9 g, PhosphonicS, Ltd.) was placed in a 5 L round bottom flask equipped with a condenser, thermowell, overhead stirrer, and sparge tube. Two (2) L of t-butanol and valencene distillate (68%, 500 g, 1.67 moles, 1 eq) were added, the contents were heated to 45° C., and the heterogeneous suspension was allowed to stir as oxygen was sparged through the solution (ca 1.5 L/min) and nitrogen flushed over the head-space. 70% t-butyl hydroperoxide in water (TBHP, 315 g, 2.45 moles, 1.47 eq) was added to the solution over 2 hr while the temperature of the reaction was heated and maintained at 60±5° C. The reaction was allowed to stir until >90% of the valencene was consumed, as determined by gas chromatography. The reaction was then allowed to cool to room temperature and the silica catalyst removed by filtration. The flask and resin were washed with 500 mL isopropanol. One (1) L of deionized water was added to the combined organic solution (t-butanol and isopropanol), and the mixture was concentrated under reduced pressure by evaporation to afford an amber colored oil. The oil was dissolved in 3 L of toluene and washed with 3.125 L of 15% sulfuric acid for 15 minutes with vigorous agitation. The aqueous layer was removed and re-extracted with 1 L of toluene. The combined toluene layers were then washed three times with 2.5 L of 1 M sodium hydroxide, twice with 500 mL saturated sodium chloride, and dried over anhydrous magnesium sulfate. After filtration, the solvent was removed under reduced pressure by evaporation to afford 378 g of viscous amber oil (33% nootkatone by weight, 0.57 moles, 34% yield).

### Example No. 4: Susceptibility of Argulus to treatment with nootkatone formulations.

This example describes an aquarium application test in which freshwater ornamental fish infected with *Argulus* were exposed to solutions of nootkatone at different concentrations to determine *Argulus* susceptibility to nootkatone.

Tests were conducted using ornamental koi carp (*Cyprinus carpio*) and gold fish (*Carassius auratus*) purchased from pet shops upon identification of *Argulus* infestation. Immediately prior to initiation of the experiment, *Argulus* infestation was confirmed macroscopically and it was noted that these crustacean ectoparasites localized mostly around the dorsal fins and anal fins.

The exposure solutions were prepared using the stock test material formulations, and tapwater as follows. Solutions were prepared within 2 hours of the start of the assay. One gram of nootkatone 98% w/w was dissolved in 1 mL of 100% ethanol to form the stock test material formulation (Solution 1) which used to produce more diluted exposure solutions. Two test concentrations (providing final exposure concentrations of 2ppm nootkatone and 6ppm nootkatone) plus a negative control (90µL ethanol comprising no nootkatone) were used.

Three clean identical glass containers were filled with tap water and into each test container were introduced three *Argulus*-infected fish (two goldfish and one koi carp per test container). The test solutions of nootkatone were added to the test containers to provide a final exposure concentration of 2ppm nootkatone and 6ppm nootkatone, and the ethanol was added to the negative control.

All fishes were observed for *Argulus* detachment after every hour.

### Results

After two hours it was noted that no parasites could be seen on the goldfish and koi carp in either test solution. Very close observation of the water revealed dead parasites at the bottom of the 2ppm and 6ppm containers. In contrast, the *Argulus* parasites in the control container remained firmly attached on the fins of gold fishes and carp fishes throughout the duration of the experiment.

### Discussion

The present study indicated 100% mortality of *Argulus* after exposure to nootkatone at concentrations of 2ppm and higher. The study also demonstrated that nootkatone is effective in an aquaculture environment, such as an aquarium. Additionally, the study complements Example 1 by confirming that nootkatone has efficacy in freshwater against fish ectoparasites.

### Sequence Listing:

Having described the invention in detail and by reference to specific embodiments thereof, it will be apparent that modifications and variations are possible without departing from the scope of the invention defined in the appended claims. More specifically, although some aspects of the present invention are identified herein as particularly advantageous, it is contemplated that the present invention is not necessarily limited to these particular aspects of the invention. Percentages disclosed herein may vary in amount by ±10, 20, or 30% from values disclosed and remain within the scope of the contemplated invention. The scope of the invention is defined by the appended claims.

## Claims

1. A composition for use in treating or preventing an infection of an ectoparasitic crustacean on a fish, comprising:
an effective concentration of nootkatone; and
an additive.

2. The composition for use of claim 1, wherein the composition is formulated for topical application to the fish, ingestion by the fish, dispersion within a pool of water, or application to the surface of aquaculture equipment.

3. The composition for use of claim 2, wherein the additive is an antibiotic or an analgesic.

4. The composition for use of claim 2, wherein the composition is formulated for ingestion by the fish and wherein the additive is a cereal, a byproduct from cereal, protein or crude protein, an oil and/or fat, a mineral, an antioxidant, a vitamin, or mixtures thereof.

5. The composition for use of claim 1 further comprising an additional active ingredient.

6. The composition of claim 1, for use in reducing rates of ectoparasitic crustacean infection and reducing incidents of bacterial or viral infection caused by an ectoparasitic crustacean infection.

7. A fish food, comprising nootkatone.

8. The fish food of claim 7, wherein the fish food comprises between 0.1 and 10 percent by weight nootkatone.

9. A composition for use in a method of reducing an ectoparasitic crustacean infection on farmed fish, the method comprising:
treating a cleaner fish species that cohabitates with the farmed fish with a composition comprising nootkatone.

10. The composition for use according to claim 9, wherein the cleaner species is from the family Labridae.

11. A composition for use in a method of preventing an ectoparasitic crustacean infection of fish, the method comprising application of a nootkatone composition to the surface of aquaculture equipment.

12. The composition for use according to claim 11, wherein the aquaculture equipment comprises one or more of a boat, a bath, a net, a fish tank liner, a float, a hose, a tarpaulin, a skirt, a filter, a pump, or a tool.

13. The composition for use according to any of the preceding claims, wherein the effective concentration of nootkatone is about 10 to about 50 mg/L, or about 15 to about 20 mg/L, or about 13.5 mg/L or, for fish that are less than about 3 cm long, between about 0.1 and about 5 ppm, wherein the term "about" refers to ±10% of any particular value.

14. The composition for use according to any of the preceding claims, wherein the ectoparasitic crustacean is a fish lice or sea lice.

15. The composition for use according to any of the preceding claims, wherein the ectoparasitic crustacean is a Branchiura, Copepoda, *Argulus*, or *Lepeophtheirus*.

## Patentansprüche

1. Zusammensetzung für die Verwendung bei der Behandlung oder Prävention einer Infektion von ektoparasitärem Krustentier auf einem Fisch, die Folgendes umfasst:
eine wirksame Konzentration von Nootkaton; und
ein Additiv.

2. Zusammensetzung für die Verwendung nach Anspruch 1, wobei die Zusammensetzung für die topische Anwendung an dem Fisch, Einnahme durch den Fisch, Dispersion innerhalb eines Wasserbeckens und Anwendung auf der Oberfläche von Aquakulturausrüstung formuliert ist.

3. Zusammensetzung für die Verwendung nach Anspruch 2, wobei das Additiv ein Antibiotikum oder ein Analgetikum ist.

4. Zusammensetzung für die Verwendung nach Anspruch 2, wobei die Zusammensetzung für die Einnahme durch den Fisch formuliert ist und wobei das Additiv Folgendes ist: ein Getreide, ein Nebenprodukt von Getreide, Protein oder Rohprotein, ein Öl und/oder Fett, ein Mineralstoff, ein Antioxidationsmittel, ein Vitamin oder Mischungen davon.

5. Zusammensetzung für die Verwendung nach Anspruch 1, die weiter einen zusätzlichen Wirkstoff umfasst.

6. Zusammensetzung nach Anspruch 1 für die Verwendung bei der Reduzierung von Infektionsraten von ektoparasitärem Krustentier und Reduzierung von Fällen von bakterieller oder viraler Infektion, die durch eine Infektion von ektoparasitärem Krustentier verursacht wurden.

7. Fischfutter, das Nootkaton umfasst.

8. Fischfutter nach Anspruch 7, wobei das Fischfutter zwischen 0,1 und 10 Gewichtsprozent Nootkaton umfasst.

9. Zusammensetzung für die Verwendung in einem Verfahren zur Reduzierung einer Infektion von ektoparasitärem Krustentier auf Zuchtfischen, wobei das Verfahren Folgendes umfasst:
Behandeln einer Putzerfischspezies, die mit den Zuchtfischen zusammenlebt, mit einer Zusammensetzung, die Nootkaton umfasst.

10. Zusammensetzung für die Verwendung nach Anspruch 9, wobei die Putzerspezies aus der Familie Labridae ist.

11. Zusammensetzung für die Verwendung in einem Verfahren zur Prävention einer Infektion von ektoparasitärem Krustentier von Fischen, wobei das Verfahren die Anwendung einer Nootkaton-Zusammensetzung auf der Oberfläche von Aquakulturausrüstung umfasst.

12. Zusammensetzung für die Verwendung nach Anspruch 11, wobei die Aquakulturausrüstung eines oder mehrere von Folgenden umfasst: ein Boot, ein Bad, ein Netz, eine Fischbeckenauskleidung, einen Schwimmer, einen Schlauch, eine Plane, eine Einfassung, einen Filter, eine Pumpe oder ein Werkzeug.

13. Zusammensetzung für die Verwendung nach einem der vorstehenden Ansprüche, wobei die wirksame Konzentration von Nootkaton etwa 10 bis etwa 50 mg/l oder etwa 15 bis etwa 20 mg/l oder etwa 13,5 mg/l oder für Fische, die weniger als etwa 3 cm lang sind, zwischen etwa 0,1 und etwa 5 ppm beträgt, wobei sich der Begriff "etwa" auf ± 10 % eines jedweden bestimmten Werts bezieht.

14. Zusammensetzung für die Verwendung nach einem der vorstehenden Ansprüche, wobei das ektoparasitäre Krustentier eine Fischlaus oder Seelaus ist.

15. Zusammensetzung für die Verwendung nach einem der vorstehenden Ansprüche, wobei das ektoparasitäre Krustentier ein Branchiura, Copepoda, *Argulus* oder *Lepeophteirus* ist.

## Revendications

1. Composition pour une utilisation destinée au traitement ou à la prévention d'une infection par un crustacé ectoparasitaire chez un poisson, comprenant
une concentration efficace en nootkatone ; et
un additif.

2. Composition pour une utilisation selon la revendication 1, la composition étant formulée pour une application topique aux poissons, une ingestion par les poissons, une dispersion au sein d'un bassin d'eau, ou une application à la surface d'équipements d'aquaculture.

3. Composition pour une utilisation selon la revendication 2, dans laquelle l'additif est un antibiotique ou un analgésique.

4. Composition pour une utilisation selon la revendication 2, la composition étant formulée pour une ingestion par les poissons, et où l'additif est une céréale, un sous-produit issu de céréales, une protéine ou une protéine brute, une huile et/ou une matière grasse, un minéral, un antioxydant, une vitamine, ou des mélanges de ceux-ci.

5. Composition pour une utilisation selon la revendication 1, comprenant en outre un ingrédient actif supplémentaire.

6. Composition selon la revendication 1, pour une utilisation destinée à la réduction des taux d'infection par des crustacés ectoparasitaires et la réduction d'incidents d'infection bactérienne ou virale provoqués par une infection par des crustacés ectoparasitaires.

7. Aliment pour poissons, comprenant de la nootkatone.

8. Aliment pour poissons selon la revendication 7, l'aliment pour poissons comprenant entre 0,1 et 10% en poids de nootkatone.

9. Composition pour une utilisation dans une méthode destinée à la réduction d'une infection par des crustacés ectoparasitaires chez des poissons d'élevage, la méthode comprenant :
le traitement d'une espèce de poisson nettoyeur qui cohabite avec le poisson d'élevage, par une composition comprenant de la nootkatone.

10. Composition pour une utilisation selon la revendication 9, l'espèce de poisson nettoyeur appartenant à la famille des *Labridae.*

11. Composition pour une utilisation dans une méthode destinée à la prévention d'une infection par des crustacés ectoparasitaires chez des poissons, la méthode comprenant l'application d'une composition à base de nootkatone à la surface d'équipements d'aquaculture.

12. Composition pour une utilisation selon la revendication 11, les équipements d'aquaculture comprenant un(e) ou plusieurs parmi un bateau, un bain, un filet, un liner pour bassin aquatique, un flotteur, un tuyau, une bâche, une jupe, un filtre, une pompe, ou un outil.

13. Composition pour une utilisation selon l'une quelconque des revendications précédentes, dans laquelle la concentration efficace en nootkatone va d'environ 10 à environ 50 mg/l, ou d'environ 15 à environ 20 mg/l, ou est d'environ 13,5 mg/l, ou, pour des poissons qui sont d'une longueur inférieure à environ 3 cm, entre environ 0,1 et environ 5 ppm, où par le terme « environ » on entend ± 10% d'une valeur particulière quelconque.

14. Composition pour une utilisation selon l'une quelconque des revendications précédentes, le crustacé ectoparasitaire étant constitué de poux des poissons ou de poux de mer.

15. Composition pour une utilisation selon l'une quelconque des revendications précédentes, le crustacé ectoparasitaire étant issu de *Branchiura, Copepoda, Argulus,* ou *Lepeophtheirus.*


### SEQUENCE LISTING

<110> Evolva SA
<120> USE OF NOOTKATONE TO TREAT SEA LICE
<130> 16-411-WO2
<160> 2
<170> PatentIn version 3.5
<210> 1
   <211> 1647
   <212> DNA
   <213> Citrus sinensis
<400> 1
   atgtcgtctg gagaaacatt tcgtcctact gcagatttcc atcctagttt atggagaaac      60
   catttcctca aaggtgcttc tgatttcaag acagttgatc atactgcaac tcaagaacga     120
   cacgaggcac tgaaagaaga ggtaaggaga atgataacag atgctgaaga taagcctgtt     180
   cagaagttac gcttgattga tgaagtacaa cgcctggggg tggcttatca ctttgagaaa     240
   gaaatagaag atgcaataca aaaattatgt ccaatctata ttgacagtaa tagagctgat     300
   ctccacaccg tttcccttca ttttcgattg cttaggcagc aaggaatcaa gatttcatgt     360
   gatgtgtttg agaagttcaa agatgatgag ggtagattca agtcatcgtt gataaacgat     420
   gttcaaggga tgttaagttt gtacgaggca gcatacatgg cagttcgcgg agaacatata     480
   ttagatgaag ccattgcttt cactaccact cacctgaagt cattggtagc tcaggatcat     540
   gtaaccccta agcttgcgga acagataaat catgctttat accgtcctct tcgtaaaacc     600
   ctaccaagat tagaggcgag gtattttatg tccatgatca attcaacaag tgatcattta     660
   tacaataaaa ctctgctgaa ttttgcaaag ttagatttta acatattgct agagctgcac     720
   aaggaggaac tcaatgaatt aacaaagtgg tggaaagatt tagacttcac tacaaaacta     780
   ccttatgcaa gagacagatt agtggagtta tatttttggg atttagggac atacttcgag     840
   cctcaatatg catttgggag aaagataatg acccaattaa attacatatt atccatcata     900
   gatgatactt atgatgcgta tggtacactt gaagaactca gcctctttac tgaagcagtt     960
   caaagatgga atattgaggc cgtagatatg cttccagaat acatgaaatt gatttacagg    1020
   acactcttag atgcttttaa tgaaattgag gaagatatgg ccaagcaagg aagatcacac    1080
   tgcgtacgtt atgcaaaaga ggagaatcaa aaagtaattg gagcatactc tgttcaagcc    1140
   aaatggttca gtgaaggtta cgttccaaca attgaggagt atatgcctat tgcactaaca    1200
   agttgtgctt acacattcgt cataacaaat tccttccttg gcatgggtga ttttgcaact    1260
   aaagaggttt ttgaatggat ctccaataac cctaaggttg taaaagcagc atcagttatc    1320
   tgcagactca tggatgacat gcaaggtcat gagtttgagc agaagagagg acatgttgcg    1380
   tcagctattg aatgttacac gaagcagcat ggtgtctcta aggaagaggc aattaaaatg 1440
   tttgaagaag aagttgcaaa tgcatggaaa gatattaacg aggagttgat gatgaagcca 1500
   accgtcgttg cccgaccact gctcgggacg attcttaatc ttgctcgtgc aattgatttt 1560
   atttacaaag aggacgacgg ctatacgcat tcttacctaa ttaaagatca aattgcttct 1620
   gtgctaggag accacgttcc attttga 1647
<210> 2
   <211> 548
   <212> PRT
   <213> Citrus sinensis
<400> 2
   Met Ser Ser Gly Glu Thr Phe Arg Pro Thr Ala Asp Phe His Pro Ser
   1 5 10 15
   Leu Trp Arg Asn His Phe Leu Lys Gly Ala Ser Asp Phe Lys Thr Val
   20 25 30
   Asp His Thr Ala Thr Gln Glu Arg His Glu Ala Leu Lys Glu Glu Val
   35 40 45
   Arg Arg Met Ile Thr Asp Ala Glu Asp Lys Pro Val Gln Lys Leu Arg
   50 55 60
   Leu Ile Asp Glu Val Gln Arg Leu Gly Val Ala Tyr His Phe Glu Lys
   65 70 75 80
   Glu Ile Glu Asp Ala Ile Gln Lys Leu Cys Pro Ile Tyr Ile Asp Ser
   85 90 95
   Asn Arg Ala Asp Leu His Thr Val Ser Leu His Phe Arg Leu Leu Arg
   100 105 110
   Gln Gln Gly Ile Lys Ile Ser Cys Asp Val Phe Glu Lys Phe Lys Asp
   115 120 125
   Asp Glu Gly Arg Phe Lys Ser Ser Leu Ile Asn Asp Val Gln Gly Met
   130 135 140
   Leu Ser Leu Tyr Glu Ala Ala Tyr Met Ala Val Arg Gly Glu His Ile
   145 150 155 160
   Leu Asp Glu Ala Ile Ala Phe Thr Thr Thr His Leu Lys Ser Leu Val
   165 170 175
   Ala Gln Asp His Val Thr Pro Lys Leu Ala Glu Gln Ile Asn His Ala
               180                 185                 190
   Leu Tyr Arg Pro Leu Arg Lys Thr Leu Pro Arg Leu Glu Ala Arg Tyr
           195                 200                 205
   Phe Met Ser Met Ile Asn Ser Thr Ser Asp His Leu Tyr Asn Lys Thr
       210                 215                 220
   Leu Leu Asn Phe Ala Lys Leu Asp Phe Asn Ile Leu Leu Glu Leu His
   225                 230                 235                 240
   Lys Glu Glu Leu Asn Glu Leu Thr Lys Trp Trp Lys Asp Leu Asp Phe
                   245                 250                 255
   Thr Thr Lys Leu Pro Tyr Ala Arg Asp Arg Leu Val Glu Leu Tyr Phe
               260                 265                 270
   Trp Asp Leu Gly Thr Tyr Phe Glu Pro Gln Tyr Ala Phe Gly Arg Lys
           275                 280                 285
   Ile Met Thr Gln Leu Asn Tyr Ile Leu Ser Ile Ile Asp Asp Thr Tyr
       290                 295                 300
   Asp Ala Tyr Gly Thr Leu Glu Glu Leu Ser Leu Phe Thr Glu Ala Val
   305                 310                 315                 320
   Gln Arg Trp Asn Ile Glu Ala Val Asp Met Leu Pro Glu Tyr Met Lys
                   325                 330                 335
   Leu Ile Tyr Arg Thr Leu Leu Asp Ala Phe Asn Glu Ile Glu Glu Asp
               340                 345                 350
   Met Ala Lys Gln Gly Arg Ser His Cys Val Arg Tyr Ala Lys Glu Glu
           355                 360                 365
   Asn Gln Lys Val Ile Gly Ala Tyr Ser Val Gln Ala Lys Trp Phe Ser
       370                 375                 380
   Glu Gly Tyr Val Pro Thr Ile Glu Glu Tyr Met Pro Ile Ala Leu Thr
   385                 390                 395                 400
   Ser Cys Ala Tyr Thr Phe Val Ile Thr Asn Ser Phe Leu Gly Met Gly
                   405                 410                 415
   Asp Phe Ala Thr Lys Glu Val Phe Glu Trp Ile Ser Asn Asn Pro Lys
               420                 425                 430
   Val Val Lys Ala Ala Ser Val Ile Cys Arg Leu Met Asp Asp Met Gln
           435                 440                 445
   Gly His Glu Phe Glu Gln Lys Arg Gly His Val Ala Ser Ala Ile Glu
       450                 455                 460
   Cys Tyr Thr Lys Gln His Gly Val Ser Lys Glu Glu Ala Ile Lys Met
   465                 470                 475                 480
   Phe Glu Glu Glu Val Ala Asn Ala Trp Lys Asp Ile Asn Glu Glu Leu
                   485                 490                 495
   Met Met Lys Pro Thr Val Val Ala Arg Pro Leu Leu Gly Thr Ile Leu
               500                 505                 510
   Asn Leu Ala Arg Ala Ile Asp Phe Ile Tyr Lys Glu Asp Asp Gly Tyr
           515                 520                 525
   Thr His Ser Tyr Leu Ile Lys Asp Gln Ile Ala Ser Val Leu Gly Asp
       530                 535                 540
   His Val Pro Phe
   545